(19) Europäisches Patentamt
European Patent Office
Office européen des brevets

(11) EP 2 445 478 B1

(12) **EUROPEAN PATENT SPECIFICATION**

(45) Date of publication and mention of the grant of the patent:
**15.05.2013 Bulletin 2013/20**

(21) Application number: **10725411.2**

(22) Date of filing: **08.06.2010**

(51) Int Cl.:
***A61K 8/81*** *(2006.01)* ***A61Q 11/00*** *(2006.01)*

(86) International application number:
**PCT/EP2010/057961**

(87) International publication number:
**WO 2010/149484 (29.12.2010 Gazette 2010/52)**

# (54) PROCESS FOR THE PRODUCTION OF A DYE-POLYMER

VERFAHREN ZUR HERSTELLUNG EINES FARBSTOFFPOLYMERS

PROCÉDÉ DE PRODUCTION D'UN POLYMÈRE COLORANT

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **26.06.2009 EP 09163936**
**22.01.2010 PCT/CN2010/000101**

(43) Date of publication of application:
**02.05.2012 Bulletin 2012/18**

(73) Proprietors:
- **Unilever PLC**
  **London EC4Y 0DY (GB)**
  Designated Contracting States:
  **CY GB IE MT**
- **Unilever NV**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO SE SI SK SM TR**

(72) Inventors:
- **BATCHELOR, Stephen Norman**
  **Wirral Merseyside CH63 3JW (GB)**
- **BIRD, Jayne Michelle**
  **Wirral Merseyside CH63 3JW (GB)**
- **CHEN, Honggang**
  **Shanghai 200335 (CN)**
- **MENG, Sheng**
  **Shanghai 200335 (CN)**
- **TAO, Qingsheng**
  **Shanghai 200335 (CN)**
- **WANG, Jinfang**
  **Shanghai 200335 (CN)**

(74) Representative: **Avila, David Victor**
**Unilever PLC**
**Unilever Patent Group**
**Colworth House**
**Sharnbrook**
**Bedford**
**Bedfordshire MK44 1LQ (GB)**

(56) References cited:
**EP-A1- 0 799 864**
**EP-A1- 2 308 932**
**WO-A1-2010/089059**
**WO-A1-2010/102861**
**DE-B- 1 217 912**
**GB-A- 567 716**
**US-A- 3 277 119**
**US-A- 4 554 091**
**US-A1- 2005 044 644**

- **ASQUITH R S ET AL: "SELF-COLOURED POLYMERS BASED ON ANTHRAQUINONE RESIDUES", JOURNAL OF THE SOCIETY OF DYERS AND COLOURISTS, SOCIETY OF DYERS AND COLOURISTS. BRADFORD, GB, vol. 93, 1 April 1977 (1977-04-01), pages 114-125, XP009048725, ISSN: 0037-9859**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 445 478 B1

## Description

The present invention concerns the provision of a method for obtaining dye polymers.

### BACKGROUND OF THE INVENTION

WO2005/003274, to Unilever, discloses that shading dyes may be included in detergent formulations to enhance the whiteness of garments.

9B567714 discloses the use of Using against in detergent products. WO09/090125 discloses cationic dyes covalently linked to polymers.

DE1217912 discloses a process for brightening textiles.

Synthesis of synthetic organic shading dye-polymers required synthesis of the dye monomer and then subsequent polymerisation reaction. It would be desirable to have a simpler synthetic route.

### SUMMARY OF THE INVENTION

The dye polymers obtained by the method of claim 1 may be used to shade textiles or provide colour cues for laundry detergent and fabric conditioners. The dye polymers are easily synthesised. The dye polymers also provide soil removal benefits.

The present invention provides a method for obtaining a dye-polymer, the method comprising the step of reacting a polymer with a primary amine of a dye to form the dye-polymer, wherein the polymer has a group for reacting with the primary amine, the group selected from: isocyanate; oxazolone; epoxide; ester, and anhydride, preferably epoxide or anhydride, most preferably anhydride.

### DETAILED DESCRIPTION THE INVENTION

### REACTION DESCRIPTION

Post-polymerization modification dye polymers are made using reactive polymers, which carry functional reactive groups that react with the $NH_2$ group of a dye.

The $NH_2$ group of a dye is reacted with the isocyanate; oxazolone, epoxide, ester, and/or anhydride of the reactive polymer. Of the ester classes, activated esters are preferred.

Post-polymerization modification using reactive polymers is described in detail in Gauthier, M.A., Gibson, M.I., and Klok H-A., Angew. Chem. Int. Ed 2009 48, 48058.

The following are examples of reacting a dye with a polymer in accordance with the present invention. Preferably the polymer does not contain a primary amine.

**Reaction (a)**

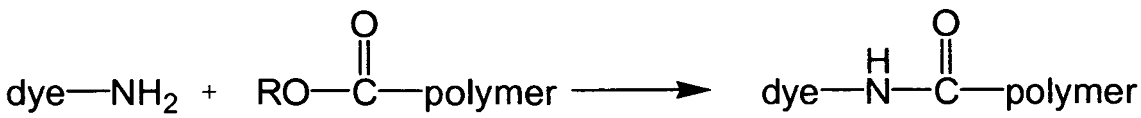

Where R is H, alkyl, aryl or selected so that the COOR group forms an anhydride.

Most preferably the reaction is of the form:

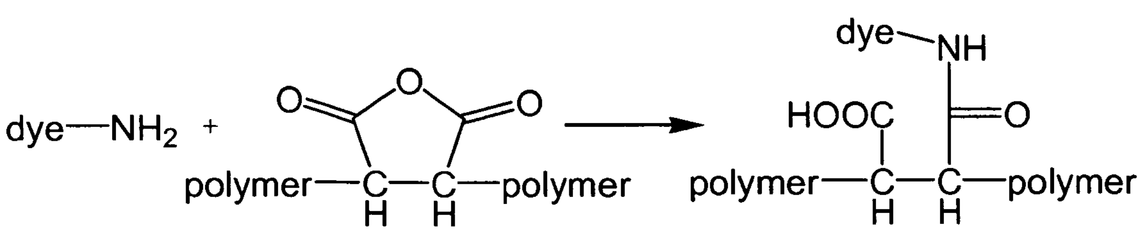

Non-limiting examples of polymers formed by such reactions are:

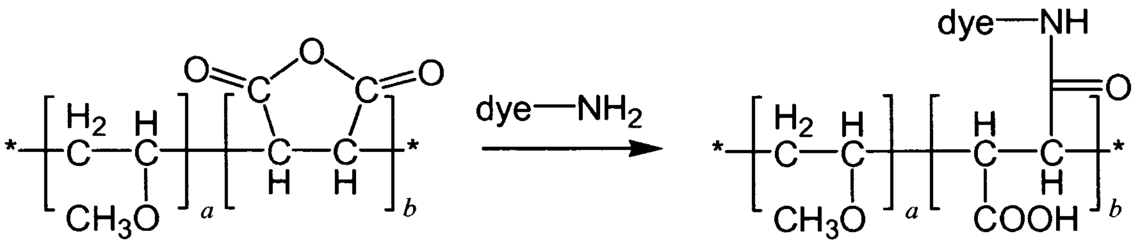

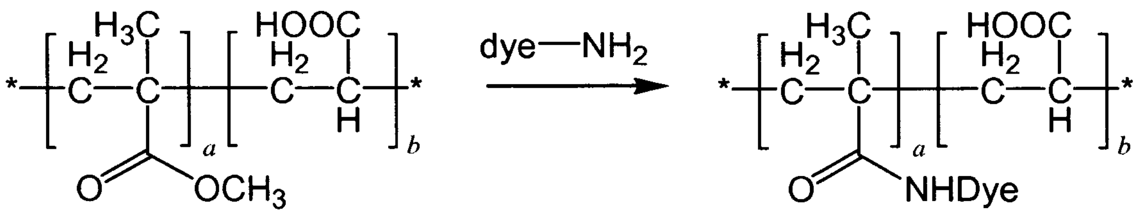

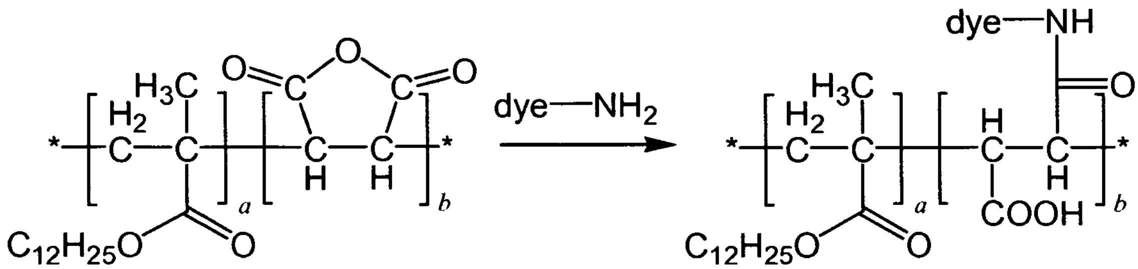

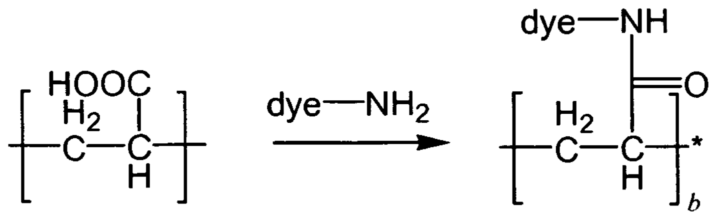

Catalysts may be used to accelerate the reactions.

Such reactions are discussed in Macromolecules 1994, 27, 7121-7126.

**Reaction (b)**

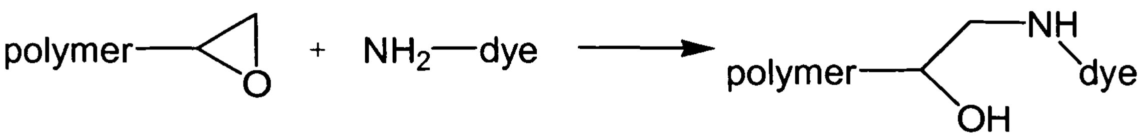

Non-limiting Examples of polymers formed by such reactions:

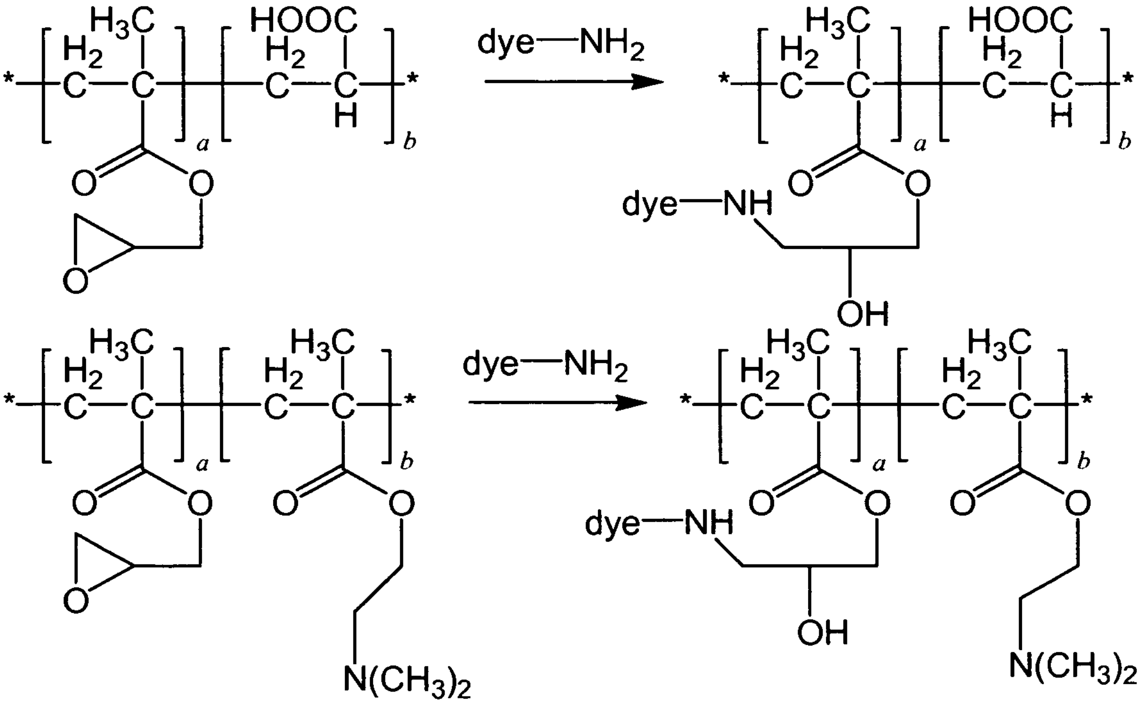


Dye description

Dyes are described in Industrial Dyes (K.Hunger ed, Wiley VCH 2003). Many dyes are listed in the colour index (Society of Dyers and Colourists and American Association of Textile Chemists and Colorists).

The dye having a primary amine may be any colour but the dye is preferably blue or violet. Preferably, the polymer is reacted with the same dye moiety. In another aspect different types of dye moieties may be reacted with the polymer, for example blue and red dyes may be reacted with the polymer to provide a good shade whilst minimising dullness. To minimise dullness whilst providing a good shade the weight ratio of the blue dye: the red dye is from 10:1 to 10:4, preferably 10:1 to 10:2.

The dye does not contain reactive groups or polymerisable double bonds. A dye containing a reactive groups is made up of a chromophore which is linked to a reactive group that undergoes addition or substitution reactions with -OH, -SH and -$NH_2$ groups to form covalent bonds. Reactive dyes are described in Industrial Dyes (K.Hunger ed, Wiley VCH 2003). Many Reactive dyes are listed in the colour index (Society of Dyers and Colourists and American Association of Textile Chemists and Colorists). Reactive groups are, for example, dichlorotriazinyl, difluorochloropyrimidine, monofluorotrazinyl, dichloroquinoxaline, vinylsulfone, difluorotriazine, monochlorotriazinyl, bromoacrlyamide and trichloropyrimidine.

Dyes are organic molecules that have an absorption coefficient of greater than 4000, preferably greater than 10 000 $mol^{-1}$ L $cm^{-1}$ at any wavelength in the range 400-700nm, preferably 540 to 640nm. Molar absorption coefficients are preferably measured in an organic solvent, preferably propan-2-ol, using a 1, 5 or 10 cm cell.

Anionic and neutral dyes are preferred. The dye is anionic and neutral in aqueous solution at a pH in the range from 4 to 10. The dyes are most preferably anionic.

The dye is preferably selected from organic dyes selected from the following chromophore classes: anthraquinone, azo, oxazine, azine, triphenodioxazine, triphenyl methane, xanthene and phthalocyanin, more preferably azo, anthraquinone and azine chromophore classes, most preferably azo and anthraquinone. The dye of preference is an anthraquinone. The dye must contain an $NH_2$ group which is covalently bound to an aromatic ring of the dye.

Preferably the dye polymer is blue or violet. Preferably the dye polymer gives a blue or violet colour to the cloth with a hue angle of 240 to 345, more preferably 265 to 330, most preferably 270 to 300. The cloth used to determine the hue angle is white bleached non-mercerised woven cotton sheeting.

Preferred dyes containing -$NH_2$ groups for such reactions are selected from: acid violet 1; acid violet 3; acid violet 6; acid violet 11; acid violet 13; acid violet 14; acid violet 19; acid violet 20; acid violet 36; acid violet 36:1; acid violet 41; acid violet 42; acid violet 43; acid violet 50; acid violet 51; acid violet 63; acid violet 48; acid blue 25; acid blue 40; acid blue 40:1; acid blue 41; acid blue 45; acid blue 47; acid blue 49; acid blue 51; acid blue 53; acid blue 56; acid blue 61; acid blue 61:1; acid blue 62; acid blue 69; acid blue 78; acid blue 81:1; acid blue 92; acid blue 96; acid blue 108; acid blue 111; acid blue 215; acid blue 230; acid blue 277; acid blue 344; acid blue 117; acid blue 124; acid blue

129; acid blue 129:1; acid blue 138; acid blue 145; direct violet 99; direct violet 5; direct violet 72; direct violet 16; direct violet 78; direct violet 77; direct violet 83; food black 2; direct blue 33; direct blue 41; direct blue 22; direct blue 71; direct blue 72; direct blue 74; direct blue 75; direct blue 82; direct blue 96; direct blue 110; direct blue 111; direct blue 120; direct blue 120:1; direct blue 121; direct blue 122; direct blue 123; direct blue 124; direct blue 126; direct blue 127; direct blue 128; direct blue 129; direct blue 130; direct blue 132; direct blue 133; direct blue 135; direct blue 138; direct blue 140; direct blue 145; direct blue 148; direct blue 149; direct blue 159; direct blue 162; direct blue 163; food black 2; food black 1 wherein the acid amide group is replaced by $NH_2$; Basic Violet 2; Basic Violet 5; Basic Violet 12; Basic Violet 14; Basic Violet 8; Basic Blue 12; Basic Blue 16; Basic Blue 17; Basic Blue 47; Basic Blue 99; disperse blue 1; disperse blue 5; disperse blue 6; disperse blue 9; disperse blue 11; disperse blue 19; disperse blue 20; disperse blue 28; disperse blue 40; disperse blue 56; disperse blue 60; disperse blue 81; disperse blue 83; disperse blue 87; disperse blue 104; disperse blue 118; disperse violet 1; disperse violet 4, disperse violet 8, disperse violet 17, disperse violet 26; disperse violet 28; solvent violet 26; solvent blue 12; solvent blue 13; solvent blue 18; solvent blue 68; and, solvent blue 76.

Further preferred dyes are selected from mono-azo dyes which contain a phenyl group directly attached to the azo group, wherein the phenyl group has an $NH_2$ groups covalent bound to it.

Examples of suitable dyes of other colours are acid red 33, acid red 30, acid red 34, acid yellow, acid green 20, acid green 33, acid brown 4, acid brown 9.

<u>POLYMER DESCRIPTION</u>

The polymer is an organic polymer which is colourless, that is to say when the polymer is dissolved in an organic solvent it has a maximum molar absorption extinction coefficient of less than 100 $mol^{-1}$ L $cm^{-1}$ in the range 400 to 700nm, preferably less than 10 $mol^{-1}$ L $cm^{-1}$.

Suitable polymers may be formed by polymerisation reaction of a monomer containing a reactive group, with further comonomers. The co-monomer does not contain primary amines.

Preferably the polymer is obtained by polymerisation of maleic anhydride or

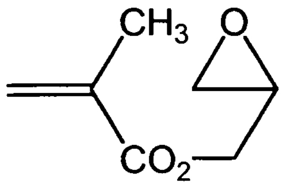

and a comonomer of the form:

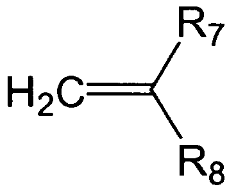

wherein $R_7$ and $R_8$ are organic groups independently selected from: H; C1-C8alkyl; OC1-C8alkyl; C(O)OH; $CO_2$C1-C18alkyl; $CO_2$C1-C4N(C1-C4a1kyl)$_2$; -C(O)N(C1-C18alkyl)$_2$; -C(O)N(C1-C18alkyl)H; -C(O)NH2; heteroaromatic; phenyl; benzyl; polyether; pyrrolidone; imidazole; cyano; Cl and F. Preferably, $R_7$ is selected from; H and CH3 and $R_8$ is selected from: -OMe and OEt.

Where C1-C18 is specified a preferred range is C1 to C4.

Further examples of co-polymer formed by polymerisation of a reactive monomer with acrylates; methacrylates; mixtures of ethyl acrylate, methyl methacrylate and methacrylic acid ; mixtures of vinylpyrrolidone with long-chain olefins; mixtures of vinylpyrrolidone and dimethylaminoethyl methacrylates; mixtures of vinylpyrrolidone and aminopropyl methacrylamides; mixtures of vinyl- pyrrolidone/aminopropyl; mixtures of vinylpyrrolidones and quaternised aminoethyl methacrylates; mixtures of vinylcaprolactam/vinylpyrrolidone/aminoethyl methacrylates; mixtured ofcaprolactam/vinyl- pyrrolidone/aminoethyl methacrylates; mixtures of styrene and acrylic acid; acrylic acids; mixtures of, co-maleic acid with unsaturated hydrocarbons; mixtures of maleic acid, vinylpyrrolidone, vinylimidazole and/or hydrophobic monomer; mixtures of vinylpyrrolidone with vinyl acetate; mixtures of maleic/acrylic acid copolymers; mixtures of lauryl methacrylate/ acrylic acid copolymers; further mixtures of the above mentioned monomers.

DYE POLYMER

Preferably, the dye polymer is blue or violet in colour. Preferably the dye polymer gives a blue or violet colour to the cloth with a hue angle of 240 to 345, more preferably 265 to 330, most preferably 270 to 300. The cloth used to determine the hue angle is white bleached non-mercerised woven cotton sheeting.

Preferably the polymer contains 0.1 to 30 Molar% dye, more preferably 1 to 15 Molar% dye monomers units, most preferably 2 to 10 Molar%.

The monomers within the polymer may be arranged in any suitable manner. For example as Alternating copolymers possess regularly alternating monomer residues; Periodic copolymers have monomer residue types arranged in a repeating sequence; Random copolymers have a random sequence of monomer residue types; Statistical copolymers have monomer residues arranged according to a known statistical rule; Block copolymers have two or more homopolymer subunits linked by covalent bonds. Most preferably the polymer is a random copolymer. The polymer should have a molecular weight 2000 and greater. Preferred ranges are from 2000 to 8000000, more preferred ranges are from 2000 to 5000000, most preferably 20000 to 3000000. In this context the molecular weight is the number average molecular weight. This is the ordinary arithmetic mean of the molecular weights of the individual macromolecules. It is determined by measuring the molecular weight of j polymer molecules, summing the weights, and dividing by j. Molecular weights are determined by Gel Permeations Chromatography.

SURFACTANT

The laundry composition comprises between 2 to 70 wt percent of a surfactant, most preferably 10 to 30 wt %. In general, the nonionic and anionic surfactants of the surfactant system may be chosen from the surfactants described "Surface Active Agents" Vol. 1, by Schwartz & Perry, Interscience 1949, Vol. 2 by Schwartz, Perry & Berch, Interscience 1958, in the current edition of "McCutcheon's Emulsifiers and Detergents" published by Manufacturing Confectioners Company or in "Tenside-Taschenbuch", H. Stache, 2nd Edn., Carl Hauser Verlag, 1981. Preferably the surfactants used are saturated.

Suitable nonionic detergent compounds which may be used include, in particular, the reaction products of compounds having a hydrophobic group and a reactive hydrogen atom, for example, aliphatic alcohols, acids, amides or alkyl phenols with alkylene oxides, especially ethylene oxide either alone or with propylene oxide. Specific nonionic detergent compounds are $C_6$ to $C_{22}$ alkyl phenol-ethylene oxide condensates, generally 5 to 25 EO, i.e. 5 to 25 units of ethylene oxide per molecule, and the condensation products of aliphatic $C_8$ to $C_{18}$ primary or secondary linear or branched alcohols with ethylene oxide, generally 5 to 40 EO.

Suitable anionic detergent compounds which may be used are usually water-soluble alkali metal salts of organic sulphates and sulphonates having alkyl radicals containing from about 8 to about 22 carbon atoms, the term alkyl being used to include the alkyl portion of higher acyl radicals. Examples of suitable synthetic anionic detergent compounds are sodium and potassium alkyl sulphates, especially those obtained by sulphating higher $C_8$ to $C_{18}$ alcohols, produced for example from tallow or coconut oil, sodium and potassium alkyl $C_9$ to $C_{20}$ benzene sulphonates, particularly sodium linear secondary alkyl $C_{10}$ to $C_{15}$ benzene sulphonates; and sodium alkyl glyceryl ether sulphates, especially those ethers of the higher alcohols derived from tallow or coconut oil and synthetic alcohols derived from petroleum. The preferred anionic detergent compounds are sodium $C_{11}$ to $C_{15}$ alkyl benzene sulphonates and sodium $C_{12}$ to $C_{18}$ alkyl sulphates. Also applicable are surfactants such as those described in EP-A-328 177 (Unilever), which show resistance to salting-out, the alkyl polyglycoside surfactants described in EP-A-070 074, and alkyl monoglycosides.

Preferred surfactant systems are mixtures of anionic with nonionic detergent active materials, in particular the groups and examples of anionic and nonionic surfactants pointed out in EP-A-346 995 (Unilever). Especially preferred is surfactant system that is a mixture of an alkali metal salt of a $C_{16}$ to $C_{18}$ primary alcohol sulphate together with a $C_{12}$ to $C_{15}$ primary alcohol 3 to 7 EO ethoxylate.

The nonionic detergent is preferably present in amounts greater than 10%, e.g. 25 to 90 wt % of the surfactant system. Anionic surfactants can be present for example in amounts in the range from about 5% to about 40 wt % of the surfactant system.

In another aspect which is also preferred the surfactant may be a cationic such that the formulation is a fabric conditioner.

CATIONIC COMPOUND

When the present invention is used as a fabric conditioner it needs to contain a cationic compound.

Most preferred are quaternary ammonium compounds.

It is advantageous if the quaternary ammonium compound is a quaternary ammonium compound having at least one $C_{12}$ to $C_{22}$ alkyl chain.

It is preferred if the quaternary ammonium compound has the following formula:

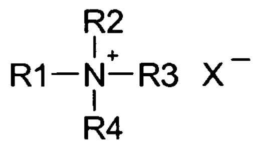

in which $R^1$ is a $C_{12}$ to $C_{22}$ alkyl or alkenyl chain; $R^2$, $R^3$ and $R^4$ are independently selected from $C_1$ to $C_4$ alkyl chains and $X^-$ is a compatible anion. A preferred compound of this type is the quaternary ammonium compound cetyl trimethyl quaternary ammonium bromide.

A second class of materials for use with the present invention are the quaternary ammonium of the above structure in which $R^1$ and $R^2$ are independently selected from $C_{12}$ to $C_{22}$ alkyl or alkenyl chain; $R^3$ and $R^4$ are independently selected from $C_1$ to $C_4$ alkyl chains and $X^-$ is a compatible anion.

Other suitable quaternary ammonium compounds are disclosed in EP 0 239 910 (Proctor and Gamble).

It is preferred if the ratio of cationic to nonionic surfactant is from 1:100 to 50:50, more preferably 1:50 to 20:50.

The cationic compound may be present from 1.5 wt % to 50 wt % of the total weight of the composition. Preferably the cationic compound may be present from 2 wt % to 25 wt %, a more preferred composition range is from 5 wt % to 20 wt %.

The softening material is preferably present in an amount of from 2 to 60% by weight of the total composition, more preferably from 2 to 40%, most preferably from 3 to 30% by weight.

The composition optionally comprises a silicone.

<u>BUILDERS OR COMPLEXING AGENTS</u>

Builder materials may be selected from 1) calcium sequestrant materials, 2) precipitating materials, 3) calcium ion-exchange materials and 4) mixtures thereof.

Examples of calcium sequestrant builder materials include alkali metal polyphosphates, such as sodium tripolyphosphate and organic sequestrants, such as ethylene diamine tetraacetic acid.

Examples of precipitating builder materials include sodium orthophosphate and sodium carbonate.

Examples of calcium ion-exchange builder materials include the various types of water-insoluble crystalline or amorphous aluminosilicates, of which zeolites are the best known representatives, e.g. zeolite A, zeolite B (also known as zeolite P), zeolite C, zeolite X, zeolite Y and also the zeolite P-type as described in EP-A-0,384,070.

The composition may also contain 0-65 % of a builder or complexing agent such as ethylenediaminetetraacetic acid, diethylenetriamine-pentaacetic acid, alkyl- or alkenylsuccinic acid, nitrilotriacetic acid or the other builders mentioned below. Many builders are also bleach-stabilising agents by virtue of their ability to complex metal ions.

Zeolite and carbonate (carbonate (including bicarbonate and sesquicarbonate) are preferred builders.

The composition may contain as builder a crystalline aluminosilicate, preferably an alkali metal aluminosilicate, more preferably a sodium aluminosilicate. This is typically present at a level of less than 15%w. Aluminosilicates are materials having the general formula:

$$0.8\text{-}1.5\ M_2O.\ Al_2O_3.\ 0.8\text{-}6\ SiO_2$$

where M is a monovalent cation, preferably sodium. These materials contain some bound water and are required to have a calcium ion exchange capacity of at least 50 mg CaO/g.
The preferred sodium aluminosilicates contain 1.5-3.5 $SiO_2$ units in the formula above. They can be prepared readily by reaction between sodium silicate and sodium aluminate, as amply described in the literature. The ratio of surfactants to alumuminosilicate (where present) is preferably greater than 5:2, more preferably greater than 3:1.

Alternatively, or additionally to the aluminosilicate builders, phosphate builders may be used. In this art the term 'phosphate' embraces diphosphate, triphosphate, and phosphonate species. Other forms of builder include silicates, such as soluble silicates, metasilicates, layered silicates (e.g. SKS-6 from Hoechst).

Preferably the laundry detergent formulation is a non-phosphate built laundry detergent formulation, i.e., contains less than 1 wt% of phosphate. Preferably the laundry detergent formulation is carbonate built.

<u>FLUORESCENT AGENT</u>

The composition preferably comprises a fluorescent agent (optical brightener). Fluorescent agents are well known and many such fluorescent agents are available commercially. Usually, these fluorescent agents are supplied

and used in the form of their alkali metal salts, for example, the sodium salts. The total amount of the fluorescent agent or agents used in the composition is generally from 0.005 to 2 wt %, more preferably 0.01 to 0.1 wt %. Preferred classes of fluorescer are: Di-styryl biphenyl compounds, e.g. Tinopal (Trade Mark) CBS-X, Di-amine stilbene di-sulphonic acid compounds, e.g. Tinopal DMS pure Xtra and Blankophor (Trade Mark) HRH, and Pyrazoline compounds, e.g. Blankophor SN. Preferred fluorescers are: sodium 2 (4-styryl-3-sulfophenyl)-2H-napthol[1,2-d]triazole, disodium 4,4'-bis{[(4-anilino-6-(N methyl-N-2 hydroxyethyl) amino 1,3,5-triazin-2-yl)]amino}stilbene-2-2' disulfonate, disodium 4,4'-bis{[(4-anilino-6-morpholino-1,3,5-triazin-2-yl)]amino} stilbene-2-2' disulfonate, and disodium 4,4'-bis(2-sulfostyryl)biphenyl.

It is preferred that the aqueous solution used in the method has a fluorescer present. When a fluorescer is present in the aqueous solution used in the method it is preferably in the range from 0.0001 g/l to 0.1 g/l, preferably 0.001 to 0.02 g/l.

PERFUME

Preferably the composition comprises a perfume. The perfume is preferably in the range from 0.001 to 3 wt %, most preferably 0.1 to 1 wt %. Many suitable examples of perfumes are provided in the CTFA (Cosmetic, Toiletry and Fragrance Association) 1992 International Buyers Guide, published by CFTA Publications and OPD 1993 Chemicals Buyers Directory 80th Annual Edition, published by Schnell Publishing Co.

It is commonplace for a plurality of perfume components to be present in a formulation. In the compositions of the present invention it is envisaged that there will be four or more, preferably five or more, more preferably six or more or even seven or more different perfume components.

In perfume mixtures preferably 15 to 25 wt% are top notes. Top notes are defined by Poucher (Journal of the Society of Cosmetic Chemists 6(2):80 [1955]). Preferred top-notes are selected from citrus oils, linalool, linalyl acetate, lavender, dihydromyrcenol, rose oxide and cis-3-hexanol.

Perfume and top note may be used to cue the whiteness benefit of the invention.

It is preferred that the laundry treatment composition does not contain a peroxygen bleach, e.g., sodium percarbonate, sodium perborate, and peracid.

OTHER POLYMERS

The composition may comprise one or more further polymers. Examples are carboxymethylcellulose, poly (ethylene glycol), poly(vinyl alcohol), polycarboxylates such as polyacrylates, maleic/acrylic acid copolymers and lauryl methacrylate/acrylic acid copolymers.

Polymers present to prevent dye deposition, for example poly(vinylpyrrolidone), poly(vinylpyridine-N-oxide), and poly(vinylimidazole), are preferably absent from the formulation.

ENZYMES

One or more enzymes are preferred present in a composition of the invention and when practicing a method of the invention.

Preferrably the level of each enzyme is from 0.0001 wt% to 0.1 wt% protein.

Especially contemplated enzymes include proteases, alpha-amylases, cellulases, lipases, peroxidases/oxidases, pectate lyases, and mannanases, or mixtures thereof.

Suitable lipases include those of bacterial or fungal origin. Chemically modified or protein engineered mutants are included. Examples of useful lipases include lipases from *Humicola* (synonym *Thermomyces),* e.g. from *H. lanuginosa (T. lanuginosus)* as described in EP 258 068 and EP 305 216 or from *H. insolens* as described in WO 96/13580, a Pseudomonas lipase, e.g. from *P. alcaligenes* or *P. pseudoalcaligenes* (EP 218 272), *P. cepacia* (EP 331 376), *P. stutzeri* (GB 1, 372, 034), *P. fluorescens,* Pseudomonas sp. strain SD 705 (WO 95/06720 and WO 96/27002). *P. wisconsinensis* (WO 96/12012), a Bacillus lipase, e.g, from *B.* subtilis (Dartois et al. (1993), Biochemica et Biophysica Acta, 1131; 253-360), or B. *pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97107202, WO 00/60063.

Preferred commercially available lipase enzymes include Lipolase™ and Lipolase Ultra™, Lipex™ (Novozymes A/S) .

The method of the invention may be carried out in the presence of phospholipase classified as EC 3.1.1.4 and/or EC 3.1.1.32. As used herein, the term phospholipase is an enzymes which has activity towards phospholipids. Phospholipids, such as lecithin or phosphatidylcholine, consist of glycerol esterified with two fatty acids in an outer (sn-1) and the middle (sn-2) positions and esterified with phosphoric acid in the third position; the phosphoric acid, in turn, may

be esterified to an amino-alcohol. Phospholipases are enzymes which participate in the hydrolysis of phospholipids, Several types of phospholipase activity can be distinguished, including phospholipases $A_1$ and $A_2$ which hydrolyze one fatty acyl group (in the sn-1 and sn-2 position, respectively) to form lysophospholipid; and lysophospholipase (or phospholipase B) which can hydrolyze the remaining fatty acyl group in lysophospholipid. Phospholipase C and phospholipase D (phosphodiesterases) release diacyl glycerol or phosphatidic acid respectively.

The enzyme and the shading dye may show some interaction and should be chosen such that this interaction is not negative. Some negative interactions may be avoided by encapsulation of one or other of enzyme or shading dye and/or other segregation within the product.

Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Preferred commercially available protease enzymes include Alcalase™, Savinase™, Primase™, Duralase™, Dyrazym™, Esperase™, Everlase™, Polarzyme™, and Kannase™, (Novozymes A/S), Maxatase™, Maxacal™, Maxapem™, Properase™, Purafect™, Purafect OxP™, FN2™, and FN3™ (Genencor International Inc.).

The method of the invention may be carried out in the presence of cutinase (classified in EC 3.1.1.74). The cutinase used according to the invention may be of any origin. Preferably cutinases are of microbial origin, in particular of bacterial, of fungal or of yeast origin.

Suitable amylases (alpha and/or beta) include those of bacterial or fungal origin. Chemically modified or protein engineered mutants are included. Amylases include, for example, alpha-amylases obtained from *Bacillus,* e.g. a special strain of *B. licheniformis,* described in more detail in GB 1,296,839, or the *Bacillus* sp. strains disclosed in WO 95/026397 or WO 00/060060. Commercially available amylases are Duramyl™, Termamyl™, Termamyl Ultra™, Natalase™, Stainzyme™, Fungamyl™ and BAN™ (Novozymes A/S), Rapidase™ and Purastar™ (from Genencor International Inc.).

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from *Humicola insolens, Thielavia terrestris, Myceliophthora thermophila,* and *Fusarium oxysporum* disclosed in US 4,435,307, US 5,648,263, US 5,691,178, US 5,776,757, WO 89/09259, WO 96/029397, and WO 98/012307. Commercially available cellulases include Celluzyme™, Carezyme™, Endolase™, Renozyme™ (Novozymes A/S), Clazinase™ and Puradax HA™ (Genencor International Inc.), and KAC-500(B)™ (Kao Corporation).

Suitable peroxidases/oxidases include those of plant, bacterial or fungal origin. Chemically modified or protein engineered mutants are included. Examples of useful peroxidases include peroxidases from *Coprinus,* e.g. from *C. cinereus,* and variants thereof as those described in WO 93/24618, WO 95/10602, and WO 98/15257. Commercially available peroxidases include Guardzyme™ and Novozym™ 51004 (Novozymes A/S).

<u>ENZYME STABILIZERS</u>

Any enzyme present in the composition may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

Where alkyl groups are sufficiently long to form branched or cyclic chains, the alkyl groups encompass branched, cyclic and linear alkyl chains. The alkyl groups are preferably linear or branched, most preferably linear.

The indefinite article "a" or "an" and its corresponding definite article "the" as used herein means at least one, or one or more, unless specified otherwise.

**<u>Example 1 Post-polymerisation modification</u>**

Reaction scheme:

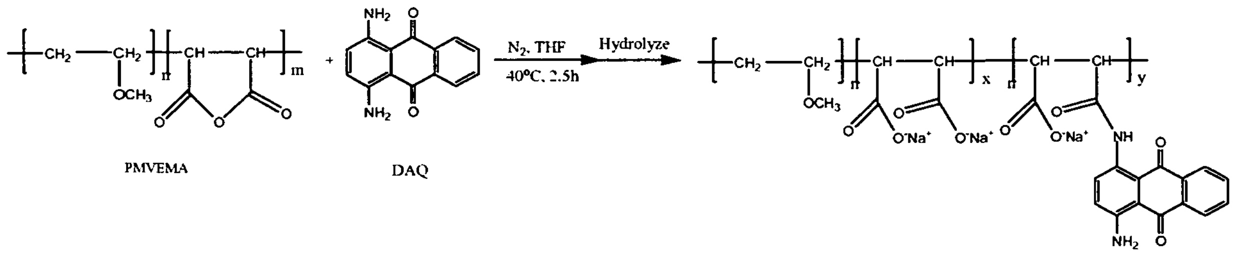


1,4-Diaminoanthraquinone (DAQ) (90% technical grade) and Poly (methyl vinyl ether-alt-maleic anhydride) (PMVEMA, CAS: 9011-16-9, Mn=80000, Mw=210000) were obtained from Aldrich and used as received. 2.044g of PMVEMA (0.013 mol maleic anhydride) and 40ml dry THF was charged into a 100ml three-necked round bottle equipped with a condenser and a magnetic stirring bar. The mixture was purged with $N_2$ stream and stirred at 40°C for 15min. Then 0.284g DAQ (0.0012 mol) dissolved in 20 ml THF was added slowly into the reactor and the reaction carried out at 65°C for 8 hours. Cooled reaction mixture was poured into 500mL petroleum ether for precipitation. The resultant polymer was further washed by $CHCl_3$ for three times to remove the non-reacted dyes and vacuum dried at r.t. over night thereafter. Finally, the resultant polymer containing non-reacted maleic anhydride underwent hydrolyzation treatment producing carbonic acid groups of moderate acidity.

The synthesis was repeated with different amounts of DAQ to give polymers with varying levels of dye substitution.

Example 2 UV-VIS of dye polymers

The UV-Vis spectra of the dye polymers of example 2 were recorded in demineralised water at 1g/L dye polymer. The UV-Vis spectra of the dye polymers of example 3 were recorded in demineralised water at 1g/L dye polymer and containing 1g/L of linear alky benzene sulphonate surfactant (LAS). The results are given in the tables below and an identifying code given to each polymer.

| Nominal inclusion level of DAQ | Code | $\lambda$max in range 400-700nm | Absorbance (1cm) @ $\lambda$max for 1g/L in water |
|---|---|---|---|
| 0.64 | P1 | 540 | 0.16 |
| 2.4 | P2 | 540 | 0.45 |
| 4.8 | P3 | 540 | 1.12 |
| 9.6 | P4 | 540 | 1.34 |

Example 3 wash deposition

Knitted white polyester (microfiber), knitted nylon-elastane (80:20) and white woven non-mercerised cotton fabrics were used together in 4g/L of a detergent which contained 15% Linear Alkyl benzene sulfonate (LAS) surfactant, 30% $Na_2CO_3$, 40% NaCl, remainder minors included calcite and fluorescer and moisture. Washes were conducted in 6° French Hard water at room temperature with a liquor to cloth ratio of 30:1, for 30 minutes. This was then repeated once more to accomplish 2 washes in total. Following the washes the cloths were rinsed twice in water, dried, their reflectance spectrum measured on a reflectometer and the colour expressed as CIE L* a* b* values (UV-excluded).

The experiment was repeated with the addition of the dye polymers of example 2. The polymers were added to give 5ppm in the wash solution. The deposition of the dye-polymers to the fabrics was expressed as the $\Delta$b value such that $\Delta$b = b(control) -b(dye polymer)

+ve values indicate a blueing of the fabric, due to dye-polymer deposition.

| Dye-polymer | $\Delta$b 4th wash | |
|---|---|---|
| | Cotton | Nylon elastane |
| P2 | 0.4 | 2.9 |
| P4 | 0.5 | 5.0 |

The dye-polymers deposit to nylon-elastane and cotton fabrics.

An added advantage is that the dye-polymer also facilitates soil removal and alter fabric feel.

Example 4 soil removal

Knitted white polyester (microfiber), white woven non-mercerised cotton fabrics and a sebum stain monitor [WFK 10 D (Cotton). (Supplied by WFK-Testgewebe GmbH, Adlerstr. 42, D-4150)] were washed together in 4g/L of a detergent which contained 15% Linear Alkyl benzene sulfonate (LAS) surfactant, 30% $Na_2CO_3$, 40% NaCl, remainder minors included calcite and fluorescer and moisture. Washes were conducted in 6° French Hard water at room temperature with a liquor to cloth ratio of 30:1, for 30 minutes. Following the washes the cloths were rinsed twice in water, dried, their reflectance spectrum measured on a reflectometer (UV-excluded).
The experiment was repeated with the addition of 1ppm in the wash solution of dye polymer P4 of example 3.
The soil removal on the WFK10D cloth was measured as the change in %Reflectance at 460nm before and after washing: $\Delta R_{460} = R_{460}$ (after wash) - $R_{460}$ (before wash).
The experiments were repeated 4 times and the average values of $\Delta R_{460}$ calculated. The results were

$$\Delta R_{460}\ \text{(control)} = 5.1$$

$$\Delta R_{460}\ \text{(P4)} = 6.2$$

The dye polymer p4 increases the soil removal.

Example 5 post-polymer modified dye polymers with anionic dye.

The reaction of example 1 was repeated using the anionic charged dye Acid Blue 62 (AB62).

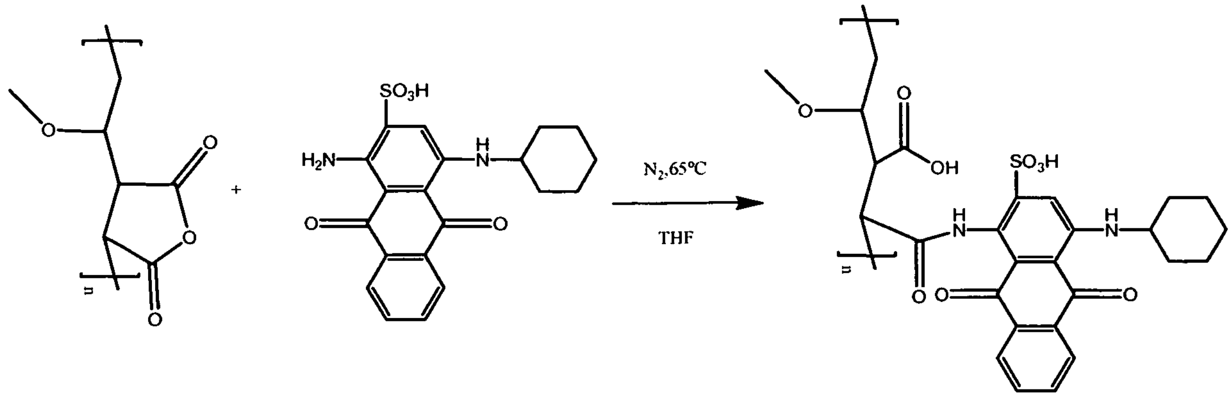


1g of the polymer was mixed with 0.1g of the dye in 50m1 of tetrahydrofuran and heated at 65°C for 8 hours. The resultant dye polymer was precipitate in petroleum ether and dried in a vacuum. The reaction was conducted twice with polymers of initial molecular weights (Mw) of 200,000 and 2,000,000 and the resultant dye polymers coded P5 and P6. Polymers were obtained from ISP (Gantrez™ AN 119 and An169, respectively).
The resultant dye polymers were blue in colour.
The wash experiment of example 3 was repeated with the current dye polymers.

| Dye-polymer | Δb 4th wash | |
|---|---|---|
| | Cotton | Nylon elastane |
| P5 | 1.0 | 2.4 |
| P6 | 0.9 | 2.6 |

Example 6 post-polymer modified dye polymers with both anionic and uncharged dyes.

The reaction of example 5 was repeated using AB62 and DAQ.

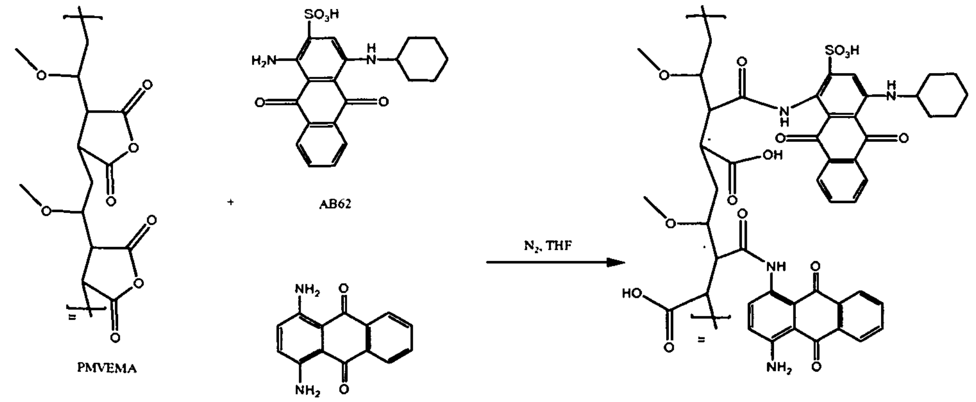


1g of the polymer (PMVEMA, CAS: 9011-16-9, Mn=80000, Mw=210000) was mixed with 0.069g acid blue 62 and 0.030 g DAQ of the dye in 50ml of tetrahydrofuran and heated at 65°C for 8 hours. The resultant dye polymer was precipitate in petroleum ether and dried in a vacuum. The resultant dye polymer was coded P7.

The wash experiment of example 3 was repeated with P7.

| Dye-polymer | Δb 4th wash | |
|---|---|---|
| | Cotton | Nylon elastane |
| P7 | 1.1 | 4.4 |

Example 7 post-polymer modified dye polymers with cationic dye.

The reaction of example 5 was repeated using the cationic azine dye MV3RAX. The dye polymers were prepared, for two a further reaction step was included, which involved binding an alkylamine to anhydride groups in a manner analogous to the dye.

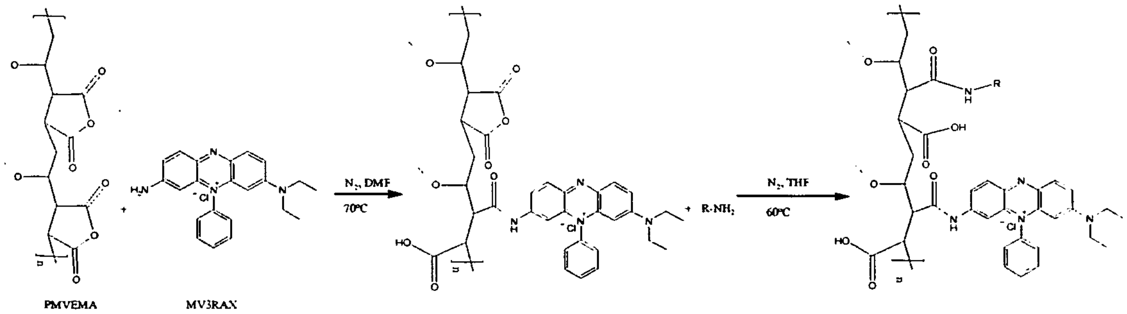


1g of the polymer (PMVEMA, CAS: 9011-16-9, Mn=80000, Mw=210000) was mixed with 0.05g MV3RAX dye in 15ml of dimethylformamide and heated at 70°C for 6 hours. The resultant dye polymer was precipitate in petroleum ether, hydrolyzed in water at 50°C and dried in a vacuum. The resultant dye polymers was coded P8.

Two further dye polymers were synthesised. Here, instead of hydrolyzing in water the dye polymer was dissolved in tetrahdyofuran and heated with 0.038g of $C_8H_{17}NH_2$ or 0.055g of $C_{12}H_{25}NH_2$ for 12 hours at 60°C. The resultant dye polymer was precipitate in petroleum ether, hydrolyzed in water at 50°C and dried in a vacuum. The polymers were coded P9 and P10 respectively.

The wash experiment of example 3 was repeated with P8, P9 and P10.

| Dye-polymer | Δb $2^{nd}$ wash |
|---|---|
| | Cotton |
| P8 | 0.5 |
| P9 | 0.3 |
| P10 | 0.3 |

## Claims

1. A method for obtaining a dye-polymer, the method comprising the step of reacting a polymer with a primary amine of a dye to form the dye-polymer, wherein the polymer has a group for reacting with the primary amine, the group selected from: isocyanate; oxazolone; epoxide; ester, and anhydride.

2. A method according to claim 1, wherein the group for reacting with the primary amine selected from: epoxide; and, anhydride.

3. A method according to claim 2, wherein the group for reacting with the primary amine is anhydride.

4. A method according to claim 1, 2 or 3, wherein the dye is selected from: anthraquinone, azo, oxazine, azine, triphenodioxazine, triphenyl methane, xanthene and phthalocyanin.

5. A method according to claim 3, wherein the dye is selected from: anthraquinone; azine; and azo,

6. A method according to claim 5, wherein the dye is selected from: anthraquinone; and azo.

7. A method according to claim 6, wherein the dye is anthraquinone.

8. A method according to any one of claims 2 to 7, wherein the polymer is obtained by polymerisation of maleic anhydride or

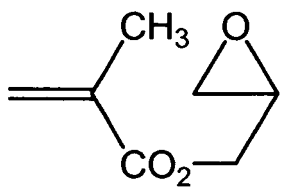

and a comonomer of the form:

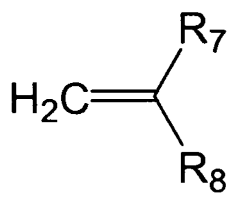

wherein $R_7$ and $R_8$ are organic groups independently selected from: H; C1-C8alkyl; OC1-C8alkyl; C(O)OH; $CO_2$C1-C18alkyl; $CO_2$C1-C4N(C1-C4alkyl)$_2$; -C(O)N(C1-C18alkyl)2; -C(O)N(C1-C18alkyl)H; -C(O)NH2; heteroaromatic; phenyl; benzyl; polyether; pyrrolidone; imidazole; cyano; Cl and F.

9. A method according to claim 8, wherein $R_7$ is selected from; H and CH3 and $R_8$ is selected from: -OMe and OEt.

10. A method according to any preceding claim 1 to 9, wherein the dye is selected from: acid violet 1; acid violet 3; acid violet 6; acid violet 11; acid violet 13; acid violet 14; acid violet 19; acid violet 20; acid violet 36; acid violet 36:1; acid violet 41; acid violet 42; acid violet 43; acid violet 50; acid violet 51; acid violet 63; acid violet 48; acid blue 25; acid blue 40; acid blue 40:1; acid blue 41; acid blue 45; acid blue 47; acid blue 49; acid blue 51; acid blue 53; acid blue 56; acid blue 61; acid blue 61:1; acid blue 62; acid blue 69; acid blue 78; acid blue 81:1; acid blue 92; acid blue 96; acid blue 108; acid blue 111; acid blue 215; acid blue 230; acid blue 277; acid blue 344; acid blue 117; acid blue 124; acid blue 129; acid blue 129:1; acid blue 138; acid blue 145; direct violet 99; direct violet 5; direct violet 72; direct violet 16; direct violet 78; direct violet 77; direct violet 83; food black 2; direct blue 33; direct blue 41; direct blue 22; direct blue 71; direct blue 72; direct blue 74: direct blue 75; direct blue 82; direct blue 96; direct blue 110; direct blue 111; direct blue 120; direct blue 120:1; direct blue 121; direct blue 122; direct blue 123; direct blue 124; direct blue 126; direct blue 127; direct blue 128; direct blue 129; direct blue 130; direct blue 132; direct blue 133;

direct blue 135; direct blue 138; direct blue 140; direct blue 145; direct blue 148; direct blue 149; direct blue 159; direct blue 162; direct blue 163; food black 2; food black 1 wherein the acid amide group is replaced by $NH_2$; Basic Violet 2; Basic Violet 5; Basic Violet 12; Basic Violet 14; Basic Violet 8; Basic Blue 12; Basic Blue 16; Basic Blue 17; Basic Blue 47; Basic Blue 99; disperse blue 1; disperse blue 5; disperse blue 6; disperse blue 9; disperse blue 11; disperse blue 19; disperse blue 20; disperse blue 28; disperse blue 40; disperse blue 56; disperse blue 60; disperse blue 81; disperse blue 83; disperse blue 87; disperse blue 104; disperse blue 118; disperse violet 1; disperse violet 4, disperse violet 8, disperse violet 17, disperse violet 26; disperse violet 28; solvent violet 26; solvent blue 12; solvent blue 13; solvent blue 18; solvent blue 68; and, solvent blue 76.

**Patentansprüche**

1. Verfahren zum Herstellen eines Farbstoffpolymers, wobei das Verfahren den Schritt des Umsetzens eines Polymers mit einem primären Amin eines Farbstoffs aufweist, um das Farbstoffpolymer zu erzeugen, wobei das Polymer eine Gruppe für die Reaktion mit dem primären Amin aufweist, wobei diese Gruppe aus Isocyanat, Oxazolon, Epoxid, Ester und Anhydrid ausgewählt ist.

2. Verfahren nach Anspruch 1, wobei die Gruppe für die Reaktion mit dem primären Amin aus Epoxid und Anhydrid ausgewählt ist.

3. Verfahren nach Anspruch 2, wobei die Gruppe für die Reaktion mit dem primären Amin Anhydrid ist.

4. Verfahren nach Anspruch 1,2 oder 3, wobei der Farbstoff aus Anthrachinon, Azo, Oxazin, Azin, Triphendioxazin, Triphenylmethan, Xanthen und Phthalocyanin ausgewählt ist.

5. Verfahren nach Anspruch 3, wobei der Farbstoff aus Anthrachinon, Azin und Azo ausgewählt ist.

6. Verfahren nach Anspruch 5, wobei der Farbstoff aus Anthrachinon und Azo ausgewählt ist.

7. Verfahren nach Anspruch 6, wobei der Farbstoff Anthrachinon ist.

8. Verfahren nach einem der Ansprüche 2 bis 7, wobei das Polymer durch Polymerisieren von Maleinsäureanhydrid oder

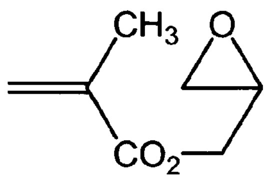

und einem Comonomer mit der Formel:

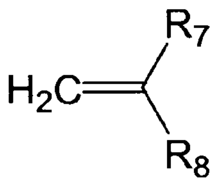

erhalten wird,
wobei $R_7$ und $R_8$ organische Reste sind, die unabhängig voneinander ausgewählt sind aus: H, $C_1$-$C_8$-Alkyl,

$OC_1$-$C_8$-alkyl, C(O)OH; $CO_2C_1$-$C_{18}$-alkyl, $CO_2C_1$-$C_4N(C_1$-$C_4$-alkyl$)_2$; -C(O)N($C_1$-$C_{18}$-alkyl$)_2$; -C(O)N($C_1$- $C_{18}$-alkyl) H, -C(O)$NH_2$, einem heteroaromatischen Rest, Phenyl, Benzyl, Polyether, Pyrrolidon, Imidazol, Cyano, Cl und F.

**9.** Verfahren nach Anspruch 8,
wobei $R_7$ aus H und $CH_3$ und $R_8$ aus -OMe und OEt ausgewählt sind.

**10.** Verfahren nach einem der vorstehenden Ansprüche 1 bis 9,
wobei der Farbstoff ausgewählt ist aus Acid Violet 1,Acid Violet 3, Acid Violet 6, Acid Violet 11, Acid Violet 13, Acid Violet 14, Acid Violet 19, Acid Violet 20, Acid Violet 36, Acid Violet 36:1, Acid Violet 41, Acid Violet 42, Acid Violet 43, Acid Violet 50, Acid Violet 51, Acid Violet 63, Acid Violet 48, Acid Blue 25, Acid Blue 40, Acid Blue 40:1, Acid Blue 41, Acid Blue 45, Acid Blue 47, Acid Blue 49, Acid Blue 51, Acid Blue 53, Acid Blue 56, Acid Blue 61, Acid Blue 61:1, Acid Blue 62, Acid Blue 69, Acid Blue 78, Acid Blue 81:1, Acid Blue 92, Acid Blue 96, Acid Blue 108, Acid Blue 111, Acid Blue 215, Acid Blue 230, Acid Blue 277, Acid Blue 344, Acid Blue 117, Acid Blue 124, Acid Blue 129, Acid Blue 129:1, Acid Blue 138, Acid Blue 145, Direct Violet 99, Direct Violet 5, Direct Violet 72, Direct Violet 16, Direct Violet 78, Direct Violet 77, Direct Violet 83, Food Black 2, Direct Blue 33, Direct Blue 41, Direct Blue 22, Direct Blue 71, Direct Blue 72, Direct Blue 74, Direct Blue 75, Direct Blue 82, Direct Blue 96, Direct Blue 110, Direct Blue 111, Direct Blue 120, Direct Blue 120:1, Direct Blue 121, Direct Blue 122, Direct Blue 123, Direct Blue 124, Direct Blue 126, Direct Blue 127, Direct Blue 128, Direct Blue 129, Direct Blue 130, Direct Blue 132, Direct Blue 133, Direct Blue 135, Direct Blue 138, Direct Blue 140, Direct Blue 145, Direct Blue 148, Direct Blue 149, Direct Blue 159, Direct Blue 162, Direct Blue 163, Food Black 2, Food Black 1, wobei die Säureamidgruppe durch $NH_2$ ersetzt ist, Basic Violet 2, Basic Violet 5, Basic Violet 12, Basic Violet 14, Basic Violet 8, Basic Blue 12, Basic Blue 16, Basic Blue 17, Basic Blue 47, Basic Blue 99, Disperse Blue 1, Disperse Blue 5, Disperse Blue 6, Disperse Blue 9, Disperse Blue 11, Disperse Blue 19, Disperse Blue 20, Disperse Blue 28, Disperse Blue 40, Disperse Blue 56, Disperse Blue 60, Disperse Blue 81, Disperse Blue 83, Disperse Blue 87, Disperse Blue 104, Disperse Blue 118, Disperse Violet 1, Disperse Violet 4, Disperse Violet 8, Disperse Violet 17, Disperse Violet 26, Disperse Violet 28, Solvent Violet 26, Solvent Blue 12, Solvent Blue 13, Solvent Blue 18, Solvent Blue 68 und Solvent Blue 76.

## Revendications

**1.** Procédé pour obtenir un colorant-polymère, le procédé comprenant l'étape de réaction d'un polymère avec une amine primaire de colorant pour former le colorant-polymère, dans lequel le polymère contient un groupe pour réagir avec l'amine primaire, le groupe étant choisi parmi : isocyanate ; oxazolone ; époxyde ; ester et anhydride.

**2.** Procédé selon la revendication 1, dans lequel le groupe pour réagir avec l'amine primaire est choisi parmi : époxyde et anhydride.

**3.** Procédé selon la revendication 2, dans lequel le groupe pour réagir avec l'amine primaire est l'anhydride.

**4.** Procédé selon les revendications 1, 2 ou 3, dans lequel le colorant est choisi parmi :

anthraquinone, azoïque, oxazine, azine, triphénodioxazine, triphénylméthane, xanthène et phtalocyanine.

**5.** Procédé selon la revendication 3, dans lequel le colorant est choisi parmi : anthraquinone, azine et azoïque.

**6.** Procédé selon la revendication 5, dans lequel le colorant est choisi parmi : anthraquinone et azoïque.

**7.** Procédé selon la revendication 6, dans lequel le colorant est l'anthraquinone.

**8.** Procédé selon l'une quelconque des revendications 2 à 7, dans lequel le polymère est obtenu par polymérisation d'anhydride maléique, ou de

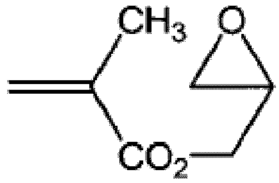

et d'un comonomère sous la forme :

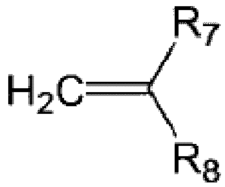

où $R_7$ et $R_8$ sont des groupes organiques indépendamment choisis parmi : H ; alkyle $C_1$-$C_8$ ; O-alkyle $C_1$-$C_8$ ; C(O)OH ; $CO_2$Cl-alkyle $C_1$-$C_{18}$ ; $CO_2$Cl-$C_4$N(alkyle $C_1$-$C_4$)$_2$ ; -C(O)N(alkyle $C_1$-$C_{18}$)$_2$ ; -C(O)N(alkyle $C_1$-$C_{18}$)H ; -C(O)$NH_2$ ; hétéroaromatique ; phényle ; benzyle ; polyéther ; pyrrolidone ; imidazole ; cyano ; Cl et F.

**9.** Procédé selon la revendication 8, dans lequel $R_7$ est choisi parmi : H et $CH_3$ et $R_8$ est choisi parmi : -OMe et OEt.

**10.** Procédé selon l'une des revendications 1 à 9 précédentes, dans lequel le colorant est choisi parmi les colorants acides "Acid Violet 1", "Acid Violet 3", "Acid Violet 6", "Acid Violet 11", "Acid Violet 13", "Acid Violet 14", "Acid Violet 19", "Acid Violet 20", "Acid Violet 36", "Acid Violet 36:1", "Acid Violet 41", "Acid Violet 42", "Acid Violet 43", "Acid Violet 50", "Acid Violet 51", "Acid Violet 63", "Acid Violet 48", "Acid Blue 25", "Acid Blue 40", "Acid Blue 40:1", "Acid Blue 41", "Acid Blue 45", "Acid Blue 47", "Acid Blue 49", "Acid Blue 51", "Acid Blue 53", "Acid Blue 56", "Acid Blue 61", "Acid Blue 61:1", "Acid Blue 62", "Acid Blue 69", "Acid Blue 78", "Acid Blue 81:1", "Acid Blue 92", "Acid Blue 96", "Acid Blue 108", "Acid Blue 111", "Acid Blue 215", "Acid Blue 230", "Acid Blue 277", "Acid Blue 344", "Acid Blue 117", "Acid Blue 124", "Acid Blue 129", "Acid Blue 129:1", "Acid Blue 138", et "Acid Blue 145" ; les colorants directs : "Direct Violet 99", "Direct Violet 5", "Direct Violet 72", "Direct Violet 16", "Direct Violet 78", "Direct Violet 77", "Direct Violet 83", le Noir alimentaire 2", les colorants directs "Direct Blue 33", "Direct Blue 41", "Direct Blue 22", "Direct Blue 71", "Direct Blue 72", "Direct Blue 74", "Direct Blue 75", "Direct Blue 82", "Direct Blue 96", "Direct Blue 110", "Direct Blue 111", "Direct Blue 120", "Direct Blue 120:1", "Direct Blue 121", "Direct Blue 122", "Direct Blue 123", "Direct Blue 124", "Direct Blue 126", "Direct Blue 127", "Direct Blue 128", "Direct Blue 129", "Direct Blue 130", "Direct Blue 132", "Direct Blue 133", "Direct Blue 135", "Direct Blue 138", "Direct Blue 140", "Direct Blue 145", "Direct Blue 148", "Direct Blue 149", "Direct Blue 159", "Direct Blue 162", et "Direct Blue 163" ; le Noir alimentaire 2 et le Noir alimentaire 1 dans lequel le groupe amide acide est remplacé par $NH_2$ ; les colorants basiques "Basic Violet 2", "Basic Violet 5", "Basic Violet 12", "Basic Violet 14", "Basic Violet 8", "Basic Blue 12", "Basic Blue 16", "Basic Blue 17", "Basic Blue 47", et "Basic Blue 99" ; les colorants dispersés "Disperse Blue 1", "Disperse Blue 5", "Disperse Blue 6", "Disperse Blue 9", "Disperse Blue 11", "Disperse Blue 19", "Disperse Blue 20", "Disperse Blue 28", "Disperse Blue 40", "Disperse Blue 56", "Disperse Blue 60", "Disperse Blue 81", "Disperse Blue 83", "Disperse Blue 87", "Disperse Blue 104", "Disperse Blue 118", "Disperse Violet 1", "Disperse Violet 4", "Disperse Violet 8", "Disperse Violet 17", "Disperse Violet 26", et "Disperse Violet 28" ; et les colorants à solvant : "Solvent Violet 26", "Solvent Blue 12", "Solvent blue 13", "Solvent blue 18", "Solvent blue 68" et "Solvent blue 76".

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005003274 A
- WO 9B567714 A
- WO 09090125 A
- DE 1217912
- EP 328177 A
- EP 070074 A
- EP 346995 A
- EP 0239910 A, Proctor and Gamble
- EP 0384070 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 97107202 A
- WO 0060063 A
- GB 1296839 A
- WO 95026397 A
- WO 00060060 A
- US 4435307 A
- US 5648263 A
- US 5691178 A
- US 5776757 A
- WO 8909259 A
- WO 96029397 A
- WO 98012307 A
- WO 9324618 A
- WO 9510602 A
- WO 9815257 A
- WO 9219709 A
- WO 9219708 A


### Non-patent literature cited in the description


- **GAUTHIER, M.A. ; GIBSON, M.I. ; KLOK H-A.** *Angew. Chem. Int. Ed,* 2009, vol. 48, 48058
- *Macromolecules,* 1994, vol. 27, 7121-7126
- Industrial Dyes. Wiley VCH, 2003
- **SCHWARTZ ; PERRY.** Surface Active Agents. Interscience, 1949, vol. 1
- **SCHWARTZ ; PERRY ; BERCH.** SURFACE ACTIVE AGENTS. Interscience, 1958, vol. 2
- McCutcheon's Emulsifiers and Detergents. Manufacturing Confectioners Company, 1981
- Tenside-Taschenbuch. Carl Hauser Verlag
- International Buyers Guide. CFTA, 1992
- OPD 1993 Chemicals Buyers Directory 80th Annual Edition. Schnell Publishing Co, 1993
- **POUCHER.** *Journal of the Society of Cosmetic Chemists,* 1955, vol. 6 (2), 80
- **DARTOIS et al.** *Biochemica et Biophysica Acta,* 1993, vol. 1131, 253-360